# EUROPÄISCHE PATENTSCHRIFT

(11) **EP 4 633 721 B1**
(45) Veröffentlichungstag und Bekanntmachung des Hinweises auf die Patenterteilung: **22.07.2026**
(21) Anmeldenummer: 23805541.2
(22) Anmeldetag: 10.11.2023
(51) Int. Cl.: A61M 39/06, A61M 39/10

(54) **VERBINDUNGSMECHANISMUS ZUR LÖSBAREN FLUIDDICHTEN VERBINDUNG VON ZWEI MEDIZINISCHEN GERÄTEN**
CONNECTION MECHANISM FOR RELEASABLE FLUID-TIGHT CONNECTION OF TWO MEDICAL APPLIANCES
MÉCANISME DE RACCORD POUR RACCORDER LIBÉRABLE DE MANIÈRE ÉTANCHE AUX FLUIDES DEUX APPAREILS MÉDICAUX

(30) Priorität: 16.12.2022 DE 102022133756
(43) Veröffentlichungstag der Anmeldung: 22.10.2025
(73) Patentinhaber: Qatna Medical GmbH, 72379 Hechingen (DE)
(72) Erfinder: SCHNEIDER, Felix, 72459 Albstadt (DE)
(74) Vertreter: DREISS Patentanwälte PartG mbB
(86) Internationale Anmeldenummer: PCT/EP2023/081384
(87) Internationale Veröffentlichungsnummer: WO 2024/125906

(56) Entgegenhaltungen:
- CN-A- 106 492 314
- US-A1- 2016 015 941
- US-A1- 2019 117 951

## Beschreibung

Die Erfindung betrifft einen Verbindungsmechanismus zur lösbaren fluiddichten Verbindung von zwei medizinischen Geräten.

Die perkutane Einführung eines medizinischen Geräts in ein Körpergefäß eines Patienten ist mit Risiken verbunden, wobei eine Vielzahl an Verfahren bekannt ist, wie das medizinische Gerät in das Gefäß oder den Körper eingeführt werden kann. Verbreitete Verfahren sind beispielsweise die Seldinger Technik.

Bei jedem dieser Verfahren muss der Fluss von Körperflüssigkeiten kontrolliert werden, wenn das medizinische Gerät in das Körpergefäß eingeführt wird. Bei Einführung in das Blutsystem ist besondere Vorsicht geboten. Bei positivem Gefäßdruck kann Blut aus dem medizinischen Gerät herausfließen. Das herausfließende Blut verschmutzt die Umgebung und kann zudem eine Infektionsgefahr für den Arzt bergen. Bei negativem Gefäßdruck besteht die Gefahr einer Luftembolie für den Patienten.

Dementsprechend werden medizinische Ventile, wie hämostatische Ventile, Irisventile, laproskopische Ports oder dergleichen verwendet, um den Blutverlust und Lufteinzug während des Eingriffs zu begrenzen oder zu verhindern.

Zudem müssen medizinische Geräte auch bei Verwendung im Körper beispielsweise mittels NaCl-Lösung gespült werden oder Medikamente werden durch sie in flüssiger Form in den Körper eingebracht, auch in diesem Anwendungsfall muss eine fluide Dichtheit Gewährleistet sein.

Einführschleusen sind selbst medizinische Geräte und werden in Verbindung mit weiteren medizinischen Geräten wie beispielsweise Dilatatoren und Kartuschen dazu genutzt chirurgische Instrumente, Implantate oder Wirkstoffe in den Körper und/oder in das Gefäßsystem eines Patienten (typischerweise eine Arterie) einzubringen. Einführschleusen sind so konzipiert, dass sie die Haut und die Wand des Blutgefäßes durchdringen und teilweise im Blutgefäß positioniert werden können, so dass chirurgische Instrumente von Außerhalb des Körpers durch die Einführschleuse eingebracht und vorgeschoben werden können, dabei ist insbesondere eine Abdichtung gegenüber der Umgebung problematisch.

Bei Verbindung zweier oder mehrerer medizinischer Geräte ist zudem die Gefahr eines Verrutschens der Geräte zueinander gegeben. Das Verrutschen kann insbesondere unabsichtlich oder durch unzureichende Sicherung der Geräte zueinander passieren. Beim Verrutschen können die Gefäße oder umliegende Gewebe verletzt werden. Zudem kann sich die Position der Geräte verändern, sodass ein Einbringen von Implantaten oder Medikamenten nicht mehr möglich ist. Weiterhin kann durch das Verrutschen eine Abdichtung zwischen den medizinischen Geräten verschlechtert bzw. geöffnet werden, sodass es zu einem ungewollten Austritt von Körperflüssigkeit kommen kann.

Die EP 2569044 B1 beschreibt eine Einführschleuse mit einem hämostatischen Ventil, das die Hämostase um chirurgische Instrumente mit einer Vielzahl von Querschnittsdurchmessern während chirurgischer Eingriffe erhalten kann.

Die US 6551283 B1 offenbart medizinische Geräte und Instrumente mit Hämostaseventilen und Hämostasekanüleneinheiten. Das Hämostaseventil weist zwei getrennte Ventilelemente, eine Ventildichtung und eine komplementär geformten Ventilmembran auf.

Die US 9616213 B2 betrifft medizinische Geräte mit verstellbaren hämostatischen Ventilen und Systemen.

In der US 9884175 B2 wird eine medizinische Ventilanordnung gezeigt, die einen Schlauch umfasst, der sich zwischen einem proximalen und einem distalen Rohrende erstreckt.

Die US 2019/0070394 A1 zeigt eine expandierbare Einführvorrichtung und Verfahren zur Verwendung derselben.

Die US 2018/0256876 A1 zeigt ein medizinisches Ventil mit einer Dichtung mit variablem Durchmesser.

In der US 2019/117951 A1 ist ein medizinischer Verbinder gezeigt. Der Verbinder umfasst eine röhrenförmige männliche Verbindereinheit, eine Eingriffseinheit, die an einer Außenseite der männlichen Verbindereinheit angeordnet ist, und einen Blockiermechanismus, der so konfiguriert ist, dass er die Verriegelungseinheit in einem vorbestimmten Zustand an der Bewegung hindert.

Die CN 106 492 314 A bezieht sich auf den Bereich der medizinischen Instrumente, insbesondere wird eine Art von Injektionsvorrichtung und Schnellkupplungsstruktur offenbart.

Die US 2016/015941 A1 zeigt eine Katheterbaugruppe.

Die Aufgabe der vorliegenden Erfindung besteht darin, einen Verbindungsmechanismus zur lösbaren fluiddichten Verbindung von zwei medizinischen Geräten mit erhöhter Sicherheit des Patienten bereit zu stellen.

Die Aufgabe wird durch einen Verbindungsmechanismus zur lösbaren fluiddichten Verbindung eines ersten

Leitungsabschnitts eines ersten medizinischen Geräts mit einem zweiten Leitungsabschnitt eines zweiten medizinischen Geräts nach Anspruch 1 gelöst. Dabei ist vorgesehen, dass der erste Leitungsabschnitt entlang einer ersten Leitungsachse verläuft und der zweite Leitungsabschnitt entlang einer zweiten Leitungsachse verläuft. Weiter ist ein am ersten Leitungsabschnitt ortsfest angeordnetes Rastelement vorgesehen, das dazu eingerichtet ist, mit einem am zweiten Leitungsabschnitt vorgesehenen Gegenrastelement in einer Verrastlage zu verrasten. Weiterhin ist ein am ersten Leitungsabschnitt verschiebbar zwischen einer Freigabelage und einer Verriegelungslage verlagerbares Verriegelungselement vorgesehen. Das Verriegelungselement ist dabei derart verlagerbar, dass es in der Verriegelungslage gegen das Rastelement wirkt, sodass das Rastelement in der Verrastlage am Gegenrastelement verriegelt ist und dass es in der Freigabelage das Rastelement freigibt, sodass das Rastelement aus der Verrastlage lösbar ist.

Durch diese Ausgestaltung kann eine sichere und definiert verbindbare sowie lösbare Verbindung zweier medizinischer Geräte erreicht werden. Ein versehentliches Verrutschen eines medizinischen Geräts zu einem zweiten medizinischen Gerät ist somit ausgeschlossen und die Sicherheit des Patienten wird gesteigert. Durch das Verbinden der Leitungsabschnitte wird zudem eine fluiddichte Verbindung erreicht aus der keine Flüssigkeit wie beispielsweise Blut, weitere Körperflüssigkeiten oder Wirkstoffe austreten können. Auch bei einem erhöhten inneren Druck in den Leitungsabschnitten bleibt die Verbindung sicher und dicht, denn durch die Verrastung des Rastelements und das Wirken des Verriegelungselements wird ein voneinander Wegbewegen der beiden Leitungsabschnitte verhindert. Zudem steigert der Verbindungsmechanismus die Sicherheit des Patienten beim Vorsehen an einer Einführschleuse und einem Dilatator insbesondere für vaskuläre Eingriffe beim Vorschieben der Einführschleuse mit dem Dilatator.

Mithilfe eines erfindungsgemäßen Verbindungsmechanismus können auch Leitungsabschnitte größerer Durchmesser als im Stand der Technik verbunden werden. Innere Durchmesser der Leitungsabschnitte von mehr als elf French können sicher und fluiddicht verbunden werden.

Erfindungsgemäß weist das Rastelement wenigstens einen schräg zur ersten Leitungsachse verlaufenden und elastisch nachgiebigen Auslenkabschnitt und einen im Wesentlichen senkrecht zur ersten Leitungsachse verlaufenden Rastabschnitt auf. Der Auslenkabschnitt ist zumindest abschnittsweise konisch ausgebildet. Der Rastabschnitt ist am freien Abschnitt des Auslenkabschnitts angeordnet, wodurch eine radiale Fläche ausgebildet wird. Der Rastabschnitt weist auch eine abschnittsweise zylindrische Fläche auf, die insbesondere parallel zur ersten Leitungsachse verlaufenden kann. Vorzugsweise sind der Auslenkabschnitt und der Rastabschnitt einstückig ausgebildet. Weiter kann das Rastelement einstückig mit dem Leitungsabschnitt ausgebildet sein oder in sonstiger Weise wie zum Beispiel stoffschlüssig durch Kleben mit dem Leitungsabschnitt verbunden sein. Ferner ist denkbar, dass der Auslenkabschnitt und oder der Rastabschnitt in Segmente unterteilt sind, welche den Leitungsabschnitt umgeben. Die elastische Nachgiebigkeit des Auslenkabschnitts ermöglicht insbesondere, dass die Verbindung des ersten und zweiten medizinischen Geräts mehrfach lösbar und verbindbar ist.

Es ist denkbar, dass das Verriegelungselement in der Verriegelungslage derart gegen den Auslenkabschnitt wirkt, dass seine elastische Nachgiebigkeit beschränkt wird. Insbesondere kann die elastische Nachgiebigkeit in radialer Richtung nach außen beschränkt werden, sodass der verrastete Zustand des Rastelements verriegelt wird. Dies stellt eine zuverlässige Art der Sicherung des Verbindungsmechanismus bereit.

Erfindungsgemäß weist das Verriegelungselement einen wenigstens abschnittsweise konisch ausgebildeten Innenkonusabschnitt auf, der in der Verriegelungslage gegen den Auslenkabschnitt wirkt. Der Innenkonus wirkt so auf die Außenseite des Auslenkabschnitts, dass die Nachgiebigkeit in radialer Richtung nach außen beschränkt wird. Die Außenseite des Bereichs des Innenkonus des Verriegelungselements kann dabei ebenfalls korrespondierend abschnittsweise konisch ausgebildet sein, um eine dünnwandige Struktur zu schaffen. Auch ein Vollkonus und/oder schräg zur Achse verlaufende Flächen als Innenseite des Verriegelungselements sind denkbar.

Vorteilhafterweise weist das Verriegelungselement einen wenigstens abschnittsweise zylindrisch ausgebildeten Betätigungsabschnitt auf. Der Betätigungsabschnitt ist dazu vorgesehen das Verriegelungselement händisch zu Verlagern. Zudem kann es Ausnehmungen für die Finger zur verbesserten Handhabung aufweisen.

Besonders bevorzugt ist ein Federelement vorgesehen, dass das Verriegelungselement hin zum Rastelement drängt. Das Verriegelungselement wird also bevorzugt in die Verriegelungslage gedrängt, wodurch es dort verbleibt. Zum Verlagern in die Freigabelage muss es entgegen einer durch das Federelement aufgebrachten Kraft verlagert werden. Denkbar ist auch, dass das Verriegelungselement von dem Rastelement weggedrängt wird, sodass es in der Freigabelage verbleibt. Insbesondere kann das Federelement als Schraubenfeder oder als Elastomer ausgebildet sein. Ferner ist denkbar, dass das Federelement einteilig mit dem Verriegelungselement ausgebildet ist. Bevorzugt ist das Federelement zwischen Betätigungsabschnitt und Leitungsabschnitt angeordnet. Durch eine Federbeaufschlagung des Verriegelungselements hin zum Rastelement wird eine zuverlässige Sicherung und definierte Positionierung der fluiddichten Verbindung erreicht.

Vorzugsweise ist der erste Leitungsabschnitt derart ausgebildet, dass er in der Verrastlage wenigstens abschnittsweise in den zweiten Leitungsabschnitt ragt. Dadurch kann die fluiddichte Verbindung zweier Leitungsabschnitte gewährleistet werden. Insbesondere kann das Ende des zweiten Leitungsabschnitts durch ein Ventilelement verschlossen sein, welches vom ersten Leitungsabschnitt durchdrungen wird und so eine fluiddichte Verbindung zur Umgebung erreicht wird.

Besonders bevorzugt umfasst der Verbindungsmechanismus ein am zweiten Leitungsabschnitt ortsfest angeordnetes Gegenrastelement, das dazu eingerichtet ist, mit dem am ersten Leitungsabschnitt vorgesehenen Rastelement in der Verrastlage zu verrasten. Bevorzugt kann das Gegenrastelement vom korrespondierend zum Rastelement ausgeführt sein. Durch die Möglichkeit der Verrastung des Rastelements in das Gegenrastelement wird eine sichere und fluiddichten Verbindung erreicht.

Das Gegenrastelement weist erfindungsgemäß einen Doppelkonus mit einem zumindest abschnittsweise konisch ausgebildeten ersten Konusabschnitt und einem zumindest abschnittsweise konisch ausgebildeten zweiten Konusabschnitt auf.

Erfindungsgemäß weitet sich der erste Konusabschnitt entlang der zweiten Leitungsachse auf einen maximalen Durchmesser auf und schließt sich der zweite Konusabschnitt an den maximalen Durchmesser an und verringert sich entlang der zweiten Leitungsachse auf einen kleineren Durchmesser. Der erste Konusabschnitt und der zweite Konusabschnitt sind bevorzugt als Konusstumpf ausgebildet. Es ist denkbar, dass der kleinste Durchmesser des zweiten Konusabschnitts größer ist, als der kleinste Durchmesser des ersten Konusabschnitts. Indem das Gegenrastelement als Doppelkonus ausgeführt ist, kann das Rastelement im Bereich des Auslenkabschnitts beim Herstellen der Verbindung elastisch verformt werden und wieder zumindest teilweise seine Ausgangsform annehmen, wenn die Verriegelungslage hergestellt ist. Ferner ist die Ausführung als Doppelkonus vorteilhaft, da eine Verbindung unabhängig der rotatorischen Lage des Lastelements und des Gegenrastelement herbeigeführt werden kann.

Es ist denkbar, dass sich an den zweiten Konusabschnitt ein zumindest teilweise zylindrisch ausgebildeter Zylinderabschnitt anschließt. Der Zylinderabschnitt eignet sich zum definierten Verrasten der zylindrischen Innenflächen des Rastabschnitts.

Bevorzugt wirkt der Rastabschnitt mit dem zweiten Konusabschnitt in der Verriegelungslage oder mit dem Zylinderabschnitt zusammen. Der zweite Konusabschnitt und oder der Zylinderabschnitt können so ausgeführt sein, dass das Rastelement in der Verrastlage elastisch verformt ist und so eine Kraft aufgebracht wird, die die Verbindung erhält.

Erfindungsgemäß liegt das Rastelement in der Verrastlage am Gegenrastelement an und die Auslenkabschnitte liegen zumindest abschnittsweise am ersten Konusabschnitt an. Durch eine definierte Anlage der Innenflächen an den ersten Konusabschnitt in der Verrastlage kann die Verbindung weiter gesichert werden, sodass auch kleinste Bewegungen zwischen dem Rastelement und dem Gegenrastelement im Gebrauch der medizinischen Geräte verhindert werden kann.

Es ist denkbar, dass ein Gehäuseabschnitt vorgesehen ist, der am zweiten Leitungsabschnitt angeordnet ist oder den zweiten Leitungsabschnitt bildet, wobei das Gegenrastelement am Gehäuseabschnitt angeordnet ist. Weiter ist denkbar, dass im Gehäuseabschnitt hämostatische Ventilelemente vorgesehen sind, wobei die Ausbildung derart ist, dass in der Verrastlage der erste Leitungsabschnitt wenigstens abschnittsweise in den zweiten Leitungsabschnitt eindringt und die hämostatischen Ventilelemente durchdringt. Ferner dichten die Ventilelemente den zweiten Leitungsabschnitt fluiddicht gegen die Umbebung ab, wenn der zweite Leitungsabschnitt nicht mit dem ersten Leitungsabschnitt verbunden ist. Die Ventilelemente können als Silikonscheiben mit sternförmigen Einschnitten oder anderen Einschnitten ausgebildet sein, wobei die Einschnitte die Dichtwirkung nicht beeinträchtigen, sondern für eine definierte Durchdringung des ersten Leitungsabschnitts sorgen. Bevorzugt sind mindestens zwei Ventilelemente im Gehäuseabschnitt vorgesehen. Weiter bevorzugt sind die Ventilelemente zueinander Verdreht um die zweite Leitungsachse angeordnet. Dies verbessert die fluiddichte Verbindung weiter.

Weitere Einzelheiten und vorteilhafte Ausgestaltungen der Erfindung sind der nachfolgenden Beschreibung zu entnehmen, anhand derer Ausführungsbeispiele der Erfindung näher beschrieben und erläutert sind.

Es zeigen:
- Figur 1:: einen ersten Leitungsabschnitt mit einem Rastelement und einem Verriegelungselement eines Verriegelungsmechanismus von der Seite;
- Figur 2:: die Anordnung der gemäß Figur 1 von hinten;
- Figur 3:: die Anordnung gemäß Figur 1 in das Rastelement hinein von vorne;
- Figur 4:: die Anordnung gemäß Figur 1 mit dem Verriegelungselement in Verriegelungslage;
- Figur 5:: die Anordnung gemäß Figur 3 mit dem Verriegelungselement in Freigabelage;
- Figur 6:: die Anordnung gemäß Figur 3 mit längs geschnittenem Rastelement;
- Figur 7:: ein Gegenrastelement des Verriegelungsmechanismus von vorne;
- Figur 8:: das Gegenrastelement geschnitten in Seitenansicht mit einem Gehäuseabschnitt und einem zweiten Leitungsabschnitt;
- Figur 9:: hämostatische Ventilelemente im Gehäuseabschnitt;
- Figur 10:: der erste Leitungsabschnitt mit dem zweiten Leitungsabschnitt verbunden mit dem Verriegelungselement in Verriegelungslage;
- Figur 11:: die Anordnung gemäß Figur 10 mit dem Verriegelungselement in Freigabelage;
- Figur 12:: das Gegenrastelement angeordnet an einer Einführschleuse;
- Figur 13:: das Rastelement angeordnet an einem Dilatator;
- Figur 14:: eine Kartusche mit dem Rastelement und dem Gegenrastelement an gegenüberliegenden Enden eines Leitungsabschnitts angeordnet; und
- Figur 15:: die Einführschleuse gemäß Figur 12 mittels des Verbindungsmechanismus verbunden mit der Kartusche gemäß Figur 14.

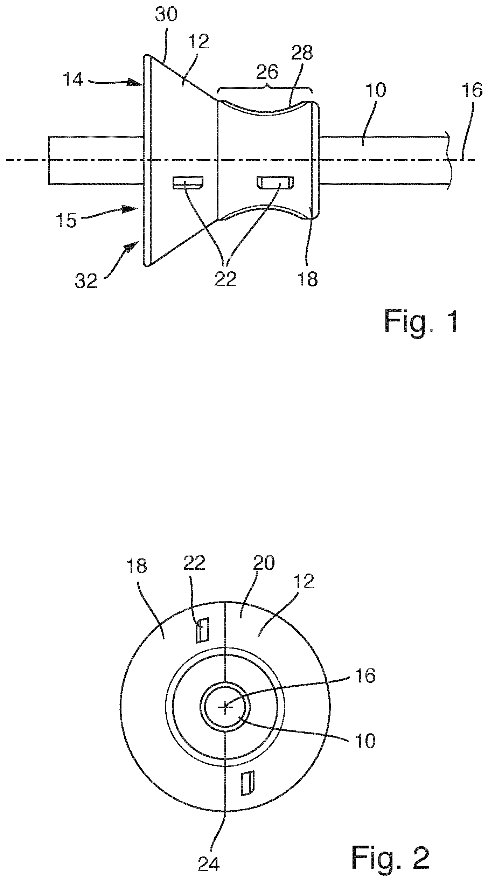

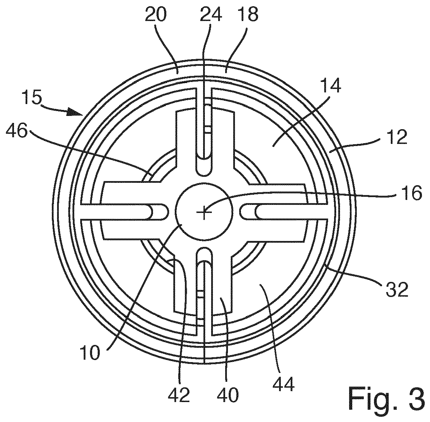

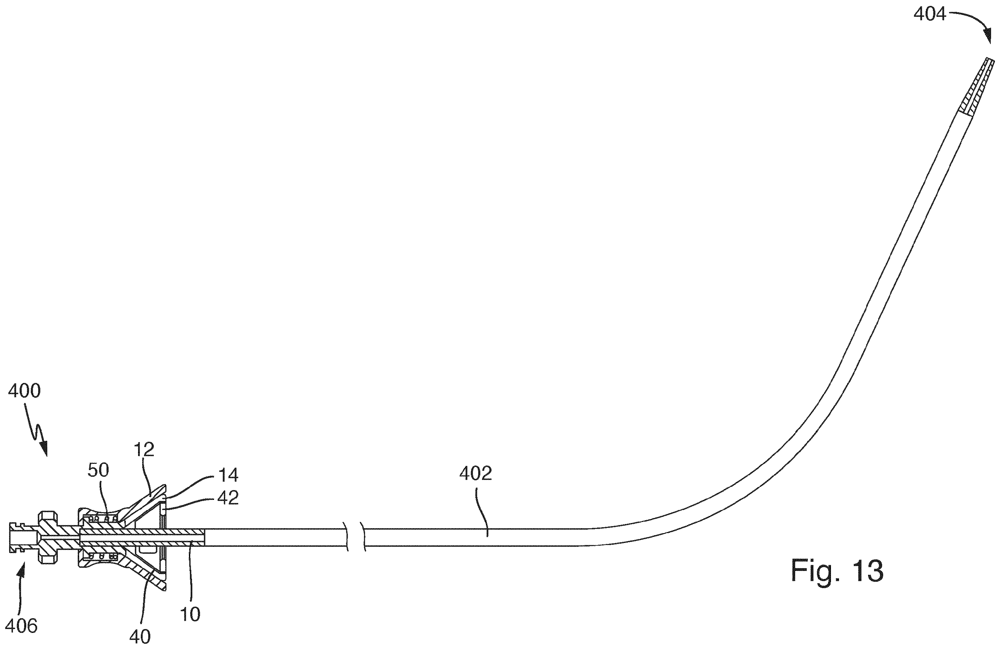

In Figur 1 und Figur 2 ist ein erster Leitungsabschnitt 10 mit einem Verriegelungselement 12 in einer Seitenansicht und einer Rückansicht dargestellt. Das Verriegelungselement 12 umgibt ein am ersten Leitungsabschnitt 10 ortsfest angeordnetes Rastelement 14, das in den Figuren 3 bis 6 in verschiedenen Ansichten gezeigt ist. Der erste Leitungsabschnitt 10 ist entlang einer ersten Leitungsachse 16 angeordnet und kann Teil eines ersten medizinischen Geräts sein, wie es beispielsweise in den Figuren 13, 14 und 15 mit den Bezugszeichen 400 und 500 gezeigt ist.

Der erste Leitungsabschnitt 10 ragt aus der Vorderseite 15 der Anordnung des Verrieglungselements 12 und Rastelements 14 hervor. Das Verriegelungselement 12 ist aus zwei verbindbaren Teilen 18 und 20 aufgebaut; es ist jedoch auch denkbar das Verriegelungselement 12 aus mehr als zwei Teilen oder einteilig aufzubauen (nicht dargestellt). Die Teile 18, 20 sind als Halbschalen ausgebildet die den Leitungsabschnitt 10 jeweils hälftig umgeben und werden mittels einer Clipsverbindung 22 formschlüssig miteinander verbunden. Zwischen den beiden Teile 18, 20 ist eine Trennstelle 24 vorgesehen, die sich in einer Ebene befindet, in der die erste Leitungsachse 16 liegt. Die Teile 18, 20 können auch stoffschlüssig durch Kleben miteinander verbunden werden (nicht gezeigt).

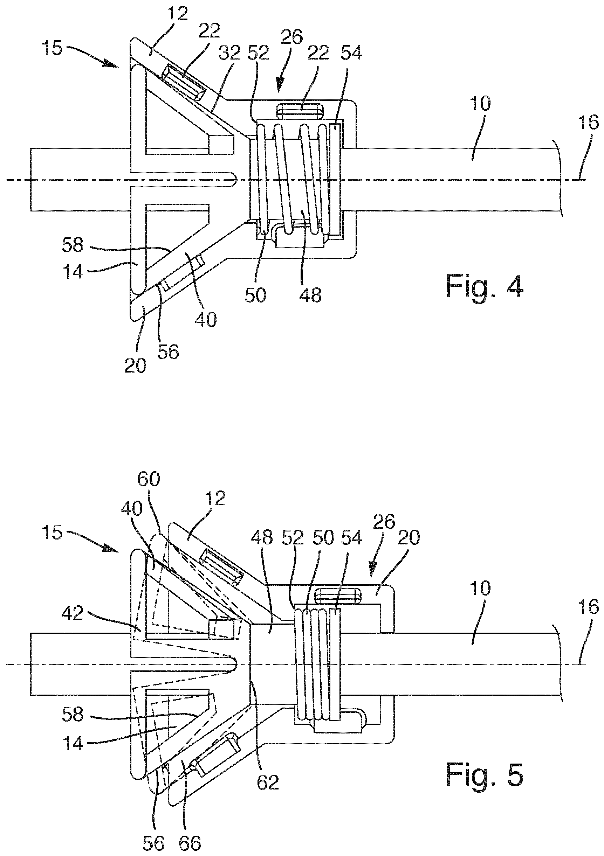

Das Verriegelungselement 12 ist entlang der Achse 16 verlagerbar. Zur händischen Verlagerung sind in einem zylindrisch ausgebildeten Betätigungsabschnitt 26 Griffmulden 28 vorgesehen. Mit diesem Griffmulden 28 kann der Bediener, z.B. ein Arzt, das Verriegelungselement 12 sicher betätigen und verlagern. Ferner ist ein äußerer konischer Abschnitt 30 vorgesehen, der korrespondierend zu einem konischen Innenabschnitt 32 ausgebildet ist, der in der Fig. 4, 5 und 6 gezeigt ist, um das Verriegelungselement 12 schlank und leicht zu gestalten und die Ergonomie zu steigern.

Figur 3 zeigt die Anordnung des ersten Leitungsabschnitts 10 mit dem Rastelement 14 und dem Verriegelungselement 12 in einer Ansicht von vorne entlang der Achse 16 in das Verriegelungselement 12 hinein. Das Rastelement 14 weist einen elastisch nachgiebigen Verformungsabschnitt 40 und einen Rastabschnitt 42 auf, der auch in den Fig. 4, 5 und 6 gezeigt ist. In dieser Ausführungsform sind der Rastabschnitt 42 und der Verformungsabschnitt 40 einstückig ausgebildet, wobei es auch denkbar wäre den Verformungsabschnitt 40 und den Rastabschnitt zweistückig und/oder aus verschiedenen Materialien auszuführen. Der Rastabschnitt 42 ist in radialer Richtung weg von der Achse 16 ausgebildet und bildet eine Fläche 44 zur Vorderseite 15 aus. Weiterhin weist der Rastabschnitt 42 eine Innenkontour 46 auf, die insbesondere lippenartig sein kann. Der Rastabschnitt 42 und der Verformungsabschnitt 40 sind in vier Segmente unterteilt, die gleichmäßig um die Achse 16 angeordnet sind. Es wäre jedoch denkbar nur ein Segment, zwei oder mehr als vier Segmente vorzusehen und/oder die Segmente nicht gleichmäßig um die Achse 16 anzuordnen.

Eine Ansicht gemäß Figur 1, jedoch ohne die Halbschale 18 des Verriegelungselements 12 ist in Figur 4 gezeigt. Das Rastelement 14 ist am ersten Leitungsabschnitt 10 angeordnet und umgeben vom Verriegelungselement 12 dargestellt. Anschließend an den Verformungsabschnitt 40 ist ein zylindrischer Abschnitt 48 angeordnet. An diesem Abschnitt 48 ist das Rastelement 14 ortsfest an dem ersten Leitungsabschnitt 10 angeordnet, dies kann insbesondere durch Kleben erfolgen. Den Abschnitt 48 umgebend ist eine Schraubenfeder 50 gezeigt. Die Schraubenfeder 50 liegt einerseits zur Vorderseite 15 an einem Anlageabschnitt 52 an dem Verriegelungselement 12 und andererseits der Vorderseite 15 abgewandt an einem umlaufenden Bund 54 am zylindrischen Abschnitt 48 des Rastelements 14 an. Dabei ist die Schraubenfeder 50 derart, dass sie das Verriegelungselement 12 gegen das Rastelement 14 in eine Verriegelungslage drängt. In der Verriegelungslage liegt der Innenabschnitt 32 zumindest teilweise an einem Außenkonusabschnitt 56 des Auslenkabschnitts 40 an und schränkt die elastische Verformbarkeit des Auslenkabschnitts 40 radial nach außen ein bzw. unterbindet diese. Ferner weist der Auslenkabschnitt 40 einen Innenkonusabschnitt 58 auf.

Figur 5 zeigt die Ansicht gemäß Figur 4 jedoch mit dem Verriegelungselement 12 in der Freigabelage. Die Schraubenfeder 50 ist komprimiert und das Verriegelungselement 12 ist entlang der Achse 16 weg von der Vorderseite 15 zurückgezogen. Anstelle der Schraubenfeder 50 sind weitere Federelemente denkbar. Beispielsweise kann ein Federelement auch einstückig mit dem Verriegelungselement 12 oder dem Rastelement 14 ausgebildet sein (nicht dargestellt). Der Innenabschnitt 32 liegt in der Freigabelage nicht am Außenkonusabschnitt 56 an, sodass der Auslenkabschnitt 40 elastisch radial nach außen entsprechend der gestrichelten Linien 60 ausgelenkt werden kann. Dazu kann der gesamte Auslenkabschnitt 40 verformt werden oder der Auslenkabschnitt 40 bildet im Bereich des Übergangs 62 in den Abschnitt 48 ein Festkörpergelenk. Es sind fensterartige Materialausnehmungen 64 im Bereich des Auslenkabschnitts 40 vorgesehen, sodass nur noch dünne Stege 66 verbleiben und diese zur Verformbarkeit beitragen. Ferner ist denkbar, statt der konischen Bereiche 32, 56, 58 und teilweise rotationssymmetrischen Geometrien des Auslenkabschnitts 40 und des Verriegelungselements 12 auch einfache korrespondierende, zur Achse 16 schräg verlaufende Flächen vorzusehen (nicht dargestellt).

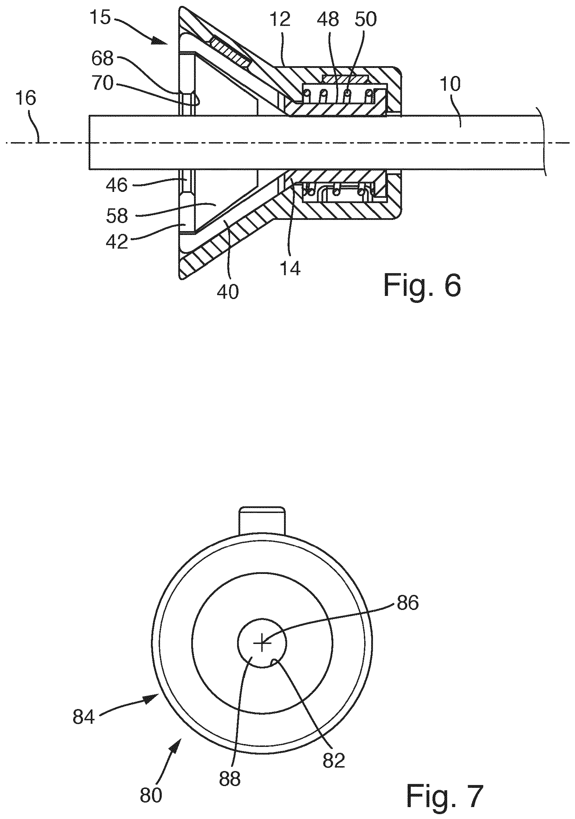

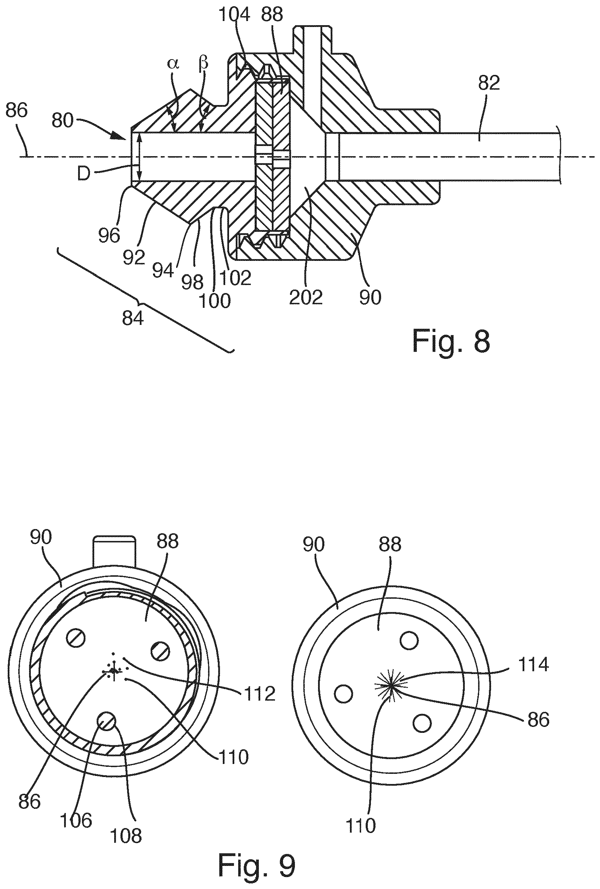

Die Figur 6 zeigt eine Ansicht gemäß Figur 4, wobei das Rastelement 14 geschnitten ist. Die Innenkontour 42 am Rastabschnitt 42 ist deutlich gezeigt. Diese ist vorzugsweise an den zur Vorderseite 15 ab und zugewandten Kanten 68, 70 abgerundet, um ein Gleiten über Flächen eines korrespondierenden Gegenrastelements 84 (in Figuren 7 und 8 gezeigt) zu verbessern.

Ein Gegenrastelement 84 ist in verschiedenen Ansichten und Schnitten in den Figuren 7 bis 9 gezeigt. Eine Ansicht auf eine Vorderseite 80 in das einen zweiten Leitungsabschnitt 82 umgebendes Gegenrastelement 84 ist in Figur 7 gezeigt. Der zweite Leitungsabschnitt 82 ist entlang einer zweiten Leitungsachse 86 ausgebildet, wobei der zweite Leitungsabschnitt 82 ein Teil eines zweiten medizinischen Geräts sein kann oder teilweise durch das medizinische Gerät und/oder durch das Gegenrastelement 84 gebildet wird.

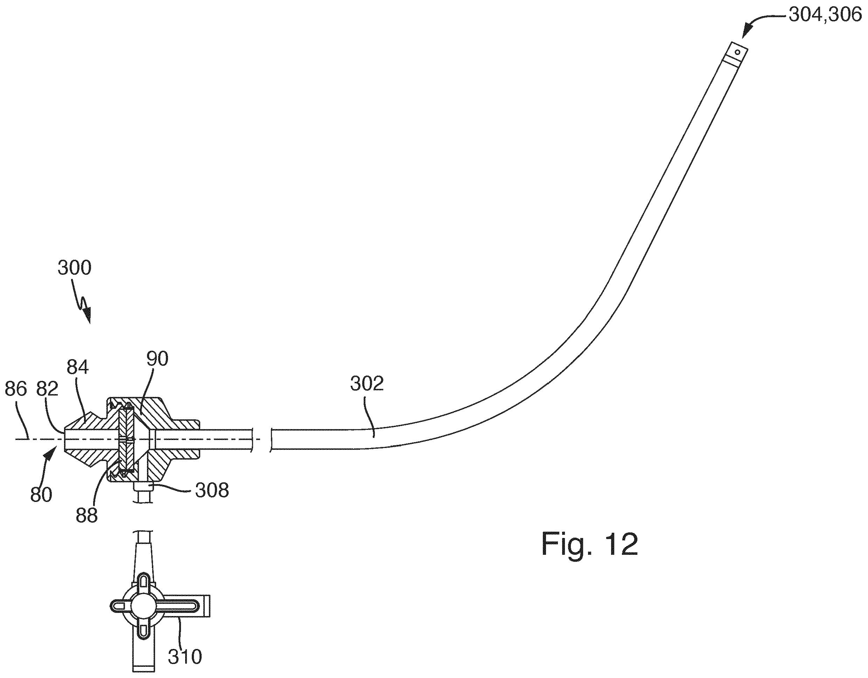

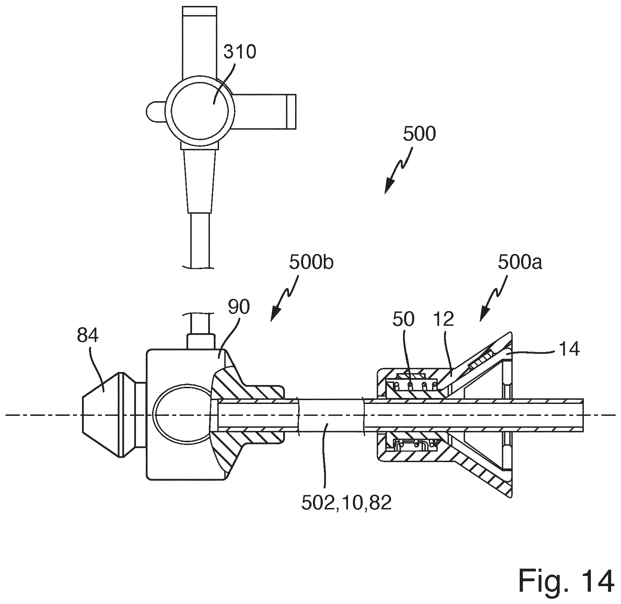

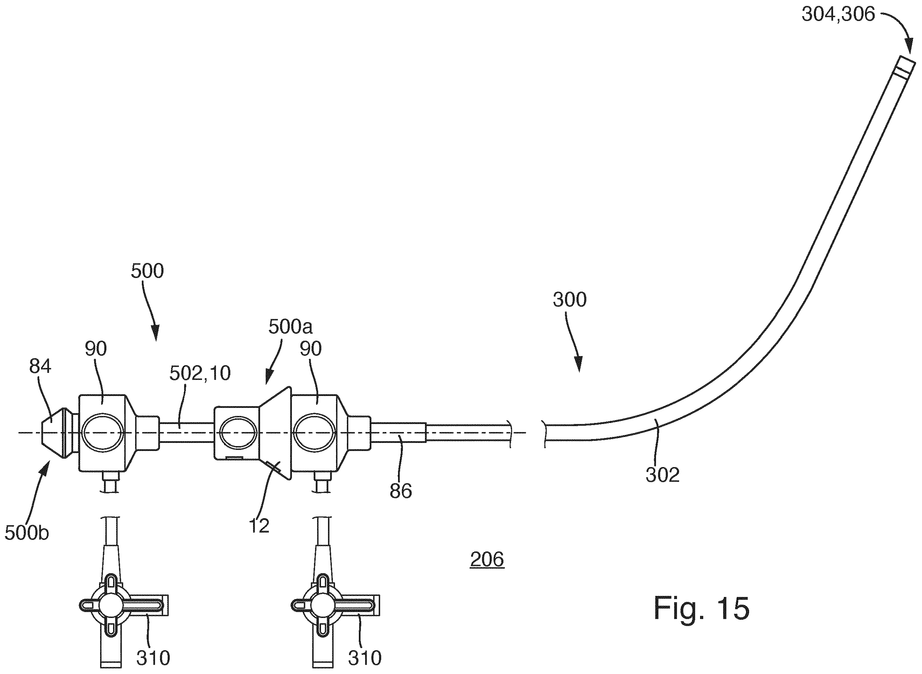

Das zweite medizinische Gerät ist in Figur 12 in Form einer Einführschleuse 300 und in Figur 14 in Form einer Kartusche 500 gezeigt. Weiter sind die Einführschleuse 300 und die Kartusche in Figur 15 miteinander verbunden gezeigt. Im Leitungsabschnitt 82 sind hämostatische Ventilelemente 88 zum abdichten des zweiten Leitungsabschnitts 82 angeordnet.

Figur 8 zeigt einen Längsschnitt durch das Gegenrastelement 84 und einem Gehäuseabschnitt 90, an dem das Gegenrastelement 84 angeordnet ist. Das Gegenrastelement 84 weist einen ersten Konusabschnitt 92 auf, der sich von der Vorderseite 80 entlang der Achse 86 vom Durchmesser D des Leitungsabschnitts 82 auf einen maximalen Durchmesser 94 auf weitet. Der minimale Durchmesser 96 des ersten Konusabschnitts 92 kann jedoch auch größer sein, als der Durchmesser **D.** Direkt an den maximalen Durchmesser 94 ist ein zweiter Konusabschnitt 98 angeordnet. Der zweite Konusabschnitt 98 verjüngt sich entlang der Achse 86 auf einen minimalen Durchmesser 100. Daran schließt sich ein zylindrischer Abschnitt 102 mit dem Durchmesser 100 an. Statt des ersten Konusabschnitts 92, des zweiten Konusabschnitts 98 und des zylindrischen Abschnitts 102 sind auch weitere nicht rotationssymmetrische Geometrien denkbar, beispielsweise schräg zur Achse 86 verlaufende Flächen (nicht dargestellt).

Das Gegenrastelement 84 ist mittels einem Verbindungselement 104 am Gehäuseabschnitt 90 anordenbar. Das Verbindungselement 104 kann insbesondere ein Gewinde sein, andere Verbdingungsarten wie beispielsweise Clipsen oder Klemmen sind denkbar. Zudem können die Ventilelemente 88 durch das Zusammensetzen, insbesondere Zusammenschrauben oder Verklemmen, des Gegenrastelements 84 mit dem Gehäuseabschnitt 90 fluiddicht verklemmt werden. Es ist mindestens ein Ventilelement 88, vorzugsweise jedoch zwei Ventilelemente 88, vorgesehen, um eine sichere Abdichtung zu gewährleisten.

Figur 9 zeigt verschiedene hämostatische Ventilelemente 88, die im Gehäuseabschnitt 90 angeordnet sind, wobei das Gegenrastelement 84 herausgeschraubt und nicht dargestellt ist. Zur verdrehsicheren Anordnung der Ventilelemente 88 sind stiftartige Elemente 106 im Gehäuseabschnitt 90 vorgesehen, die mit entsprechenden Löchern 108 in den Ventilelementen 88 korrespondieren. In den Ventilelementen 88 sind selbst dichtende Durchgänge 110 im Bereich des zweiten Leitungsabschnitts 82 um die Achse 86 vorgesehen, die ein fluiddichtes Hindurchführen des ersten Leitungsabschnitts 10 oder anderer medizinischer Geräte 400, 500a ermöglichen. Die Einschnitte können als Perforationen 112 oder sternförmige Einschnitte 114 ausgebildet sein. Weiterhin können die Ventilelemente 88 entsprechend der Achse 86 versetzt angeordnet werden, um eine weiter verbesserte Abdichtung zu gewährleisten.

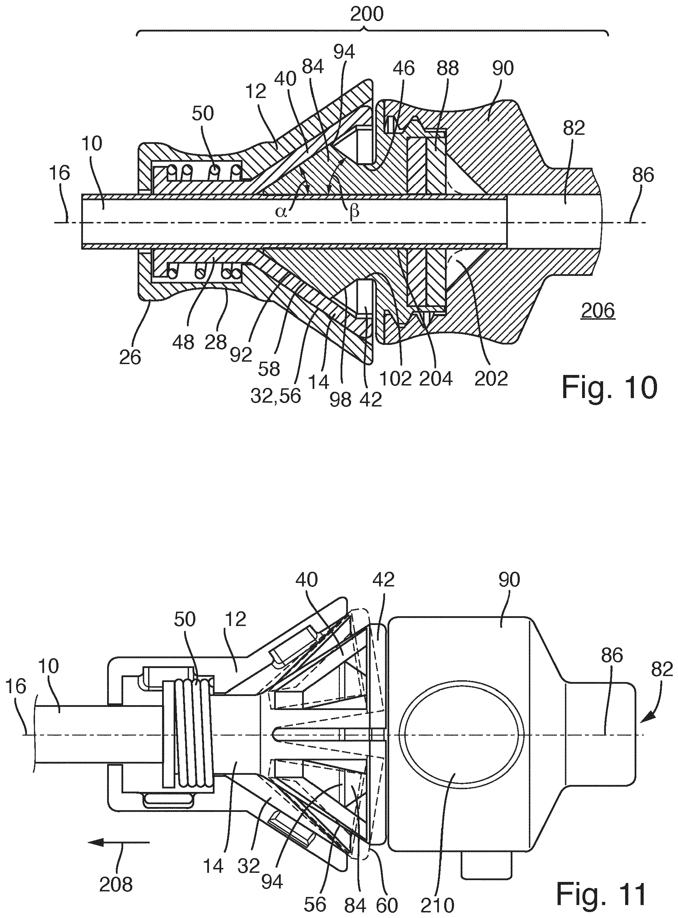

Figur 10 zeigt einen Längsschnitt mit dem ersten Leitungsabschnitt 10, der mit dem zweiten Leitungsabschnitt 82 durch den Verbindungsmechanismus 200 fluiddicht verbunden ist. Das Rastelement 14 befindet sich mit dem Gegenrastelement 84 in der Verrastlage. Der Innenkonusabschnitt 58 liegt am ersten Konusabschnitt 92 an. Der Rastabschnitt 42 liegt mit der Innenkontour 46 am zylindrischen Abschnitt 102 an. Der Verformungsabschnitt 40 kann in der Verrastlage verformt sein, da der Durchmesser 100 größer sein kann als der Durchmesser der Innenkontour 46. Das Verriegelungselement 12 wird durch die Feder 50 gegen den Außenkonusabschnitt 56 des Verformungsabschnitts 40 gedrängt, sodass eine weitere Verformung des Verformungsabschnitts 40 nicht möglich ist. Die Verbindung ist somit nicht lösbar und lässt keine Bewegung entlang der Achsen 16, 86 relativ zwischen den Leitungsabschnitten 10, 82 zu.

Aufgrund des zumindest teilweise rotationssymmetrischen Aufbaus des Rastelements 14, des Gegenrastelements 84 und des Verriegelungselements 12 können die Komponenten 12, 14, 84, relativ zueinander um die Achsen 16, 86 verdreht werden, auch dann, wenn sich der Verbindungsmechanismus 200 in Verrastlage befindet und die Verbindung hergestellt ist. Auch bei einem Verdrehen bleibt die Verbindung fluiddicht. Dies ist dann vorteilhaft, wenn die Position im eingeführten Zustand in den Körper verändert werden muss. Ferner ist das Herstellen und Lösen der Verbindung 200 unabhängig der rotatorischen Position um die Achsen 16, 86 möglich. Die erste Leitungsachse 16 ist im Bereich des Verbindungsmechanismus 200 deckungsgleich mit der zweiten Leitungsachse 86. Der erste Leitungsabschnitt 10 ragt in den zweiten Leitungsabschnitt 82 hinein und durchdringt die Ventilelemente 88, diese können durchdrungen und/oder in einen Hohlraum 202 verdrängt werden. Dadurch wird die Abdichtung verbessert und das einführen des ersten Leistungsabschnitts 10 vereinfacht. Die Ventilelemente 88 liegen an der Außenseite 204 des ersten Leitungsabschnitts 10 mit den Durchgängen 110 zumindest teilweise an und dichten die Verbindung zur Umgebung 206 ab. Es könnte ein zweiter Hohlraum (nicht dargestellt) auf der gegenüberliegenden Seite des Hohlraums 202, der Vorderseite 80 zugewandt, vorgesehen sein. Dieser zweite Hohlraum würde das Herausziehen des ersten Leitungsabschnitts 10 beim Trennen der Verbindung vereinfachen und verringert eine Abnutzung der Ventilelemente 88.

In Figur 11 ist die Ansicht gemäß Figur 10 gezeigt, jedoch mit dem Verriegelungselement 12 in die Freigabelage verlagert. Die Feder 50 ist komprimiert. Zum Lösen der Verbindung muss das Verriegelungselement in Pfeilrichtung 208 in die Freigabelage aus der Verriegelungslage entsprechend der Darstellung in Figur 10 verlagert werden. Zum Ziehen des Verriegelungselements eignen sich die Griffmulden 28 und/oder der Betätigungsabschnitt 26. Dadurch wird das Rastelement 12 freigegeben. Wird weiter in Richtung 208 gezogen, so wird der Auslenkabschnitt 40 durch den Flächenkontakt der Innenkontour 46 und zweitem Konusabschnitt 98 radial nach außen entsprechend der Linien 60 elastisch ausgelenkt und die Verbindung wird aus der Verrastlage heraus gelöst. Sobald der Rastabschnitt 42 mit der Innenkontour 46 den maximalen Durchmesser 94 überwunden hat, nimmt der Auslenkabschnitt 40 wieder seine Ausgangslage entsprechend Figur 4 an. Wird weiter in Richtung 208 gezogen, so gleitet der erste Leitungsabschnitt 10 aus dem zweiten Leitungsabschnitt 82 und den Ventilelementen 88 heraus und die Verbindung wird getrennt. Der zweite Leitungsabschnitt 88 ist im getrennten Zustand durch die Ventilelemente 88 gegen die Umgebung 206 abgedichtet.

Für eine weiter verbesserte Handhabung sind am Gehäuseabschnitt 90 auch Griffmulden 210 vorgesehen. Dies ist vorteilhaft, wenn der Gehäuseabschnitt 90 an einem medizinischen Gerät angeordnet ist oder ein solches bildet und es in den Körper eingeführt ist und beim Trennen der Verbindung nicht herausgezogen werden soll. Zum Herstellen der Verbindung muss zunächst der erste Leitungsabschnitt 10 entgegen der Richtung 208 in den zweiten Leitungsabschnitt 86 im Gegenrastelement 84 eingeführt werden, dann muss das Verriegelungselement 12 in Pfeilrichtung 208 in die Freigabelage zurückgezogen werden, sodass die Verformbarkeit des Auslenkabschnitts 40 ermöglicht wird. Der Auslenkabschnitt 40 wird durch den ersten Konusabschnitt 92 verformt und durch den zweiten Konusabschnitt 98 zumindest teilweise bis in die Verrastlage entformt. Sobald das Rastelement 14 in der Verrastlage ist, kann das Verriegelungselement 12 losgelassen werden, sodass die Feder 50 es gegen das Rastelement 14 in die Verriegelungslage drängt und dieses wie beschrieben verriegelt. Zur weiteren Verbesserung der Handhabung des Verbindungsmechanismus 200 ist denkbar, dass die Anordnung von Verriegelungselement 12, Rastelement 14 und Feder 50 derart aufeinander abgestimmt ist, dass das Verriegelungselement 12 zum Herstellen der Verbindung nicht zurückgezogen werden muss. Das Aufschieben des Rastelements 14 auf den ersten Konusabschnitt 92 verursacht in solch einer Ausführung sowohl eine Verformung des Rastelements 14 und ein verlagern des Verriegelungselements 12, sodass ein vorheriges zurückziehen des Verriegelungselements 12 nicht nötig ist. Zum Trennen der Verbindung muss das Verriegelungselement 12 jedoch zurückgezogen werden. Dies kann durch unterschiedliche Winkel α des ersten Konusabschnitts 92 und Winkel β des zweiten Konusabschnitts 98 in Bezug auf die Achse 86 erreicht werden.

Figur 12 zeigt ein zweites medizinisches Gerät in Form einer Einführschleuse 300 mit dem Gehäuseabschnitt 90, mit den Ventilelementen 88 und dem Gegenrastelement 84. Der zweite Leitungsabschnitt 82 wird teilweise vom Gegenrastelement 84 und dem Gehäuseabschnitt 90 gebildet. Der Vorderseite 80 des Gegenrastelements 84 abgewandt ist am Gehäuseabschnitt 90 ein Einführrohr 302 angeordnet, welches den Leitungsabschnitt 86 bis zu seinem Ende 304 fortsetzt. Der Leitungsabschnitt 86 mündet am freien Ende 304 in eine Öffnung 306. Das Einführrohr 302 kann verschiedene Längen und Innendurchmesser je nach Zweck aufweisen. Ferner kann es für den gewünschten Einsatzzweck vorgeformt und steif oder flexibel ausgeführt sein. Im Gehäuseabschnitt 90 ist ein Abzweig 308 vom Leitungsabschnitt 86 vorgesehen, an dem ein drei-wege Hahn 310 angeordnet ist. Mittels des drei-wege Hahns 310 kann der Leitungsabschnitt 86 gespült, Wirkstoffe eingebracht, Flüssigkeiten abgesaugt und aspiriert werden.

In Figur 13 ist ein erstes medizinisches Gerät in Form eines Dilatators 400 mit einem Dilatatorkörper 402 und mit einer Spitze 404, um einen Kanal im Gewebe beim Einführen in einen Körper aufzuweiten. Das Rastelement 14 und das Verriegelungselement 12 sind am Dilatatorkörper 402 angeordnet, wobei der Dilatatorkörper 402 den ersten Leitungsabschnitt 10 bildet. Ferner ist ein Lueranschluss 406 am Rastelement 14 angeordnet, welches den Leitungsabschnitt 10 fortsetzt. Der Leitungsabschnitt 10 ist vom Lueranschluss 406 bis zur Spitze 404 durchgängig ausgebildet. Am Lueranschluss 406 kann eine Spritze zum Injizieren, insbesondere zum Spülen, angeschlossen werden (nicht dargestellt). Weiterhin kann in den Leitungabschnitt 10 des Dilatators 300 ein Führungsdraht (nicht dargestellt) zum Einführen in einen Körper eingeführt werden.

Der Dilatator 400 eignet sich dazu mit der Einführschleuse 300 mittels des Verbindungsmechanismus 200 verbunden zu werden, um die Einführschleuse 300 sicher in einen Körper einzuführen. Der Dilatator 400 verhindert auch ein Abknicken des Einführrohrs 302 beim Einführen in den Körper. Nach dem Einführen kann der Dilatator 300 sicher von der Einführschleuse 300 mittels des Verbindungsmechanismus 200 getrennt und herausgezogen werden, ohne, dass dabei Körperflüssigkeiten austreten und/oder die Gefahr einer Luftembolie besteht.

Figur 14 zeigt eine Kartusche 500, die ein erstes medizinisches Gerät 500a und ein zweites medizinisches Gerät 500b umfasst. Das eine Ende bildet das erste medizinische Gerät 500a aus und das andere Ende bildet das zweite medizinisches Gerät 500b aus, wobei sowohl das Rastelement 14, das Verriegelungselement 12, das Gegenrastelement 84 und der Gehäuseabschnitt 90 an einem gemeinsamen Leitungsabschnitt 502 angeordnet sind. Für den Leitungsabschnitt 502 gilt im Bereich des Rastelements 14 das für den Leitungsabschnitt 10 gesagte und im Bereich des Gehäuseabschnitts 90 und Gegenrastelements 84 das für den Leitungsabschnitt 82 gesagte. Die Kartusche 500 eignet sich dazu mit der Einführschleuse 300 mittels des Verbindungsmechanismus 200 verbunden zu werden. Die Kartusche 500 kann im Leitungsabschnitt 502 Wirkstoffe oder Implantate aufnehmen, welche mittels der Einführschleuse 300 an eine bestimmte anatomische Position in den Körper eingebracht werden können. Eine derartige Kartusche 500 mit Komponenten des Verbindungsmechanismus 200 ermöglicht es noch weitere medizinische Geräte wie eine weitere Kartusche 500 zu verbinden und so die Einführschleuse 300 fluiddicht gegen die Umgebung 206 zu erweitern.

Figur 15 zeigt die Kartusche 500, die mittels des Verbindungsmechanismus 200 mit der Einführschleuse 300 verbunden ist. Das Verriegelungselement 14 befindet sich in der Verriegelungslage. Der gemeinsame Leitungsabschnitt 502 kann als erster Leitungsabschnitt 10 gesehen werden und ist mit dem zweiten Leitunsabschnitt 86 fluiddicht und sicher verbunden. Zum Lösen der Verbindung gilt das zu den Figuren 10 und 11 gesagte. Es ist erkennbar, dass am freien Rastelement 84 am proximalen Ende der Kartusche 500 am zweiten medizinischen Gerät 500b ein weiteres erste medizinisches Gerät 500a, 400, aufweisend eine Anordnung aus Rastelement 14, Verriegelungselement 12 und Leitungsabschnitt 10 anordenbar ist. Denkbar ist auch, dass zwei oder mehr Kartuschen 500 miteinander verbindbar und hintereinander anordenbar sind.

Mit dem gezeigten Verbindungsmechanismus 200 ist es möglich verschiedene medizinische Geräte, wie eine Einführschleuse 300, einen Dilatator 400 und/oder eine Kartusche 500 fluiddicht und sicher miteinander zu Verbinden und die Verbindung wieder zu lösen. Weiterhin ist die Verbindung unabhängig der rotatorischen Position der medizinischen Geräte bezüglich ihrer Achsen 10, 86, 502 herstellbar, lösbar und/oder die medizinischen Geräte zueinander verdrehbar. Dies steigert die Sicherheit des Patienten, bei dem medizinische Geräte 300, 400, 500 mit dem Verbindungsmechanismus 200 eingesetzt werden.

## Patentansprüche

1. Verbindungsmechanismus (200) zur lösbaren fluiddichten Verbindung eines entlang einer ersten Leitungsachse (16) verlaufenden ersten Leitungsabschnitts (10) eines ersten medizinischen Geräts (400, 500a) mit einem entlang einer zweiten Leitungsachse (86) verlaufenden zweiten Leitungsabschnitt (82) eines zweiten medizinischen Geräts (300, 500b),
der Verbindungsmechanismus (200) aufweisend einen entlang einer ersten Leitungsachse (16) verlaufenden ersten Leitungsabschnitt (10) mit einem am ersten Leitungsabschnitt (10) ortsfest
angeordneten Rastelement (14),
und einen entlang einer zweiten Leitungsachse (86) verlaufenden zweiten Leitungsabschnitt (82) mit einem Gegenrastelement (84), wobei das Rastelement (14) dazu eingerichtet ist, mit dem am zweiten Leitungsabschnitt (82) vorgesehenen Gegenrastelement (84) in einer Verrastlage zu verrasten,
der Verbindungsmechanismus(200) weiterhin aufweisend ein am ersten Leitungsabschnitt (10) verschiebbar zwischen einer Freigabelage und einer Verriegelungslage verlagerbares Verriegelungselement (12) derart,
dass das Verriegelungselement (12) in der Verriegelungslage gegen das Rastelement (14) wirkt, sodass das Rastelement (14) in der Verrastlage am Gegenrastelement (84) verriegelt ist und dass das Verriegelungselement (12) in der Freigabelage das Rastelement (14) freigibt, sodass das Rastelement (14) aus der Verrastlage lösbar ist,
wobei das Rastelement (14) wenigstens einen zumindest abschnittsweise konisch ausgebildeten, schräg zur ersten Leitungsachse (16) verlaufenden und elastisch nachgiebigen Auslenkabschnitt (40) und einen im Wesentlichen senkrecht zur ersten Leitungsachse (16) verlaufenden Rastabschnitt (42) aufweist,
wobei das Verriegelungselement (12) einen wenigstens abschnittsweise konisch ausgebildeten Innenkonusabschnitt (32) aufweist, der in der Verriegelungslage gegen den Auslenkabschnitt (40) wirkt, wobei das Gegenrastelement (84) einen Doppelkonus mit einem zumindest abschnittsweise konisch ausgebildeten ersten Konusabschnitt (92) und einem zumindest abschnittsweise konisch ausgebildeten zweiten Konusabschnitt (98) aufweist,
wobei sich der erste Konusabschnitt (92) entlang der zweiten Leitungsachse (86) auf einen maximalen Durchmesser aufweitet und sich der zweite Konusabschnitt (98) an den maximalen Durchmesser anschließt und sich entlang der zweiten Leitungsachse (86) auf einen kleineren Durchmesser verringert, und
wobei in der Verrastlage das Rastelement (14) am Gegenrastelement (84) anliegt und der Auslenkabschnitt (40) zumindest abschnittsweise am ersten Konusabschnitt (92) anliegt.

2. Verbindungsmechanismus (200) nach Anspruch **1,** wobei das Verriegelungselement (12) in der Verriegelungslage derart gegen den Auslenkabschnitt (40) wirkt, dass seine elastische Nachgiebigkeit beschränkt wird.

3. Verbindungsmechanismus (200) nach einem der vorherigen Ansprüche, wobei das Verriegelungselement (12) einen wenigstens abschnittsweise zylindrisch ausgebildeten Betätigungsabschnitt (26) aufweist.

4. Verbindungsmechanismus (200) nach einem der vorherigen Ansprüche, wobei ein Federelement (50) vorgesehen ist, das das Verriegelungselement (12) hin zum Rastelement (14) drängt.

5. Verbindungsmechanismus (200) nach Anspruch 4, wobei der erste Leitungsabschnitt (10) derart ausgebildet ist, dass er in der Verrastlage wenigstens abschnittsweise in den zweiten Leitungsabschnitt (82) ragt.

6. Verbindungsmechanismus (200) nach einem der vorhergehenden Ansprüche, mit einem am zweiten Leitungsabschnitt (82) ortsfest angeordneten Gegenrastelement (84), das dazu eingerichtet ist, mit dem am ersten Leitungsabschnitt (10) vorgesehenen Rastelement (14) in der Verrastlage zu verrasten.

7. Verbindungsmechanismus (200) nach einem der vorhergehenden Ansprüche, wobei sich an den zweiten Konusabschnitt (98) ein zumindest teilweise zylindrisch ausgebildeter Zylinderabschnitt (102) anschließt.

8. Verbindungsmechanismus (200) nach einem der vorhergehenden Ansprüche, wobei in der Verriegelungslage der Rastabschnitt (42) mit dem zweiten Konusabschnitt (98) zusammenwirkt.

9. Verbindungsmechanismus (200) nach Anspruch 7, wobei in der Verriegelungslage der Rastabschnitt (42) mit dem Zylinderabschnitt (102) zusammenwirkt.

10. Verbindungsmechanismus (200) nach einem der vorhergehenden Ansprüche, wobei ein Gehäuseabschnitt (90) vorgesehen ist, der am zweiten Leitungsabschnitt (82) angeordnet ist oder den zweiten Leitungsabschnitt (82) bildet, wobei das Gegenrastelement (84) am Gehäuseabschnitt (90) angeordnet ist, wobei im Gehäuseabschnitt (90) hämostatische Ventilelemente (88) vorgesehen sind, und wobei die Ausbildung derart ist, dass in der Verrastlage der erste Leitungsabschnitt (10) wenigstens abschnittsweise in den zweiten Leitungsabschnitt (82) eindringt und die hämostatischen Ventilelemente (88) durchdringt.

## Claims

1. A connection mechanism (200) for releasable fluid-tight connection of a first line portion (10), extending along a first line axis (16), of a first medical appliance (400, 500a) to a second line portion (82), extending along a second line axis (86), of a second medical appliance (300, 500b), the connection mechanism (200) comprising
a first line portion (10) which extends along a first line axis (16) and has an engagement element (14), which is arranged so as to be stationary on the first line portion (10),
and a second line portion (82) which extends along a second line axis (86) and has a counter-engagement element (84), wherein the engagement element (14) is designed to engage with the counter-engagement element (84), provided on the second line portion (82), in an engagement position,
the connection mechanism (200) further comprising a locking element (12), which can be displaced on the first line portion (10) and is movable between a release position and a locking position such that,
in the locking position, the locking element (12) acts counter to the engagement element (14) so that, in the engagement position, the engagement element (14) is locked on the counter-engagement element (84) and such that, in the release position, the locking element (12) releases the engagement element (14) so that the engagement element (14) can be released from the engagement position,
wherein the engagement element (14) has at least one resiliently flexible deflection portion (40) which is at least partially conical and extends obliquely to the first line axis (16) and an engagement portion (42) which extends substantially perpendicularly to the first line axis (16),
wherein the locking element (12) has an inner cone portion (32) which is at least partially conical and acts against the deflection portion (40) in the locking position,
wherein the counter-engagement element (84) has a double cone comprising a first cone portion (92) which is at least partially conical and a second cone portion (98) which is at least partially conical,
wherein the first cone portion (92) widens to a maximum diameter along the second line axis (86) and the second cone portion (98) adjoins the maximum diameter and decreases to a smaller diameter along the second line axis (86), and
wherein, in the engagement position, the engagement element (14) rests against the counter-engagement element (84) and the deflection portion (40) rests at least partially against the first cone portion (92).

2. The connection mechanism (200) according to claim 1, wherein, in the locking position, the locking element (12) acts against the deflection portion (40) in such a way that its resilient flexibility is limited.

3. The connection mechanism (200) according to any of the preceding claims, wherein the locking element (12) has an actuating portion (26) which is at least partially cylindrical.

4. The connection mechanism (200) according to any of the preceding claims, wherein a spring element (50) is provided that urges the locking element (12) toward the engagement element (14).

5. The connection mechanism (200) according to claim 4, wherein the first line portion (10) is designed such that in the engagement position, it protrudes at least partially into the second line portion (82).

6. The connection mechanism (200) according to any of the preceding claims, with a counter-engagement element (84) which is arranged so as to be stationary on the second line portion (82) and is designed to engage with the engagement element (14) provided on the first line portion (10) in the engagement position.

7. The connection mechanism (200) according to any of the preceding claims, wherein an at least partially cylindrical cylinder portion (102) adjoins the second cone portion (98).

8. The connection mechanism (200) according to any of the preceding claims, wherein, in the locking position, the engagement portion (42) interacts with the second cone portion (98).

9. The connection mechanism (200) according to claim 7, wherein, in the locking position, the engagement portion (42) interacts with the cylinder portion (102).

10. The connection mechanism (200) according to any of the preceding claims, wherein a housing portion (90) is provided which is arranged on the second line portion (82) or forms the second line portion (82), wherein the counter-engagement element (84) is arranged on the housing portion (90), wherein hemostatic valve elements (88) are provided in the housing portion (90), and wherein the design is such that, in the engagement position, the first line portion (10) penetrates at least partially into the second line portion (82) and penetrates the hemostatic valve elements (88).

## Revendications

1. Mécanisme de connexion (200) destiné à connecter de manière étanche aux fluides et détachable une première section de ligne (10) d'un premier appareil médical (400, 500a), s'étendant le long d'un premier axe de ligne (16), à une deuxième section de ligne (82) d'un deuxième appareil médical (300, 500b), s'étendant le long d'un deuxième axe de ligne (86), ledit mécanisme de connexion (200) comprenant
une première section de ligne (10) s'étendant le long d'un premier axe de ligne (16) et ayant un élément à enclenchement (14) disposé de manière fixe sur la première section de ligne (10),
et une deuxième section de ligne (82) s'étendant le long d'un deuxième axe de ligne (86) et ayant un contre-élément à enclenchement (84), dans lequel ledit élément à enclenchement (14) est conçu pour s'enclencher dans une position d'enclenchement avec le contre-élément à enclenchement (84) prévu sur la deuxième section de ligne (82),
le mécanisme de connexion (200) comprenant en outre un élément de verrouillage (12) qui peut être déplacé à coulissement sur la première section de ligne (10) entre une position de libération et une position de verrouillage de telle sorte
qu'en position de verrouillage, ledit élément de verrouillage (12) agit contre l'élément à enclenchement (14) de sorte que l'élément à enclenchement (14) soit verrouillé sur le contre-élément à enclenchement (84) en position d'enclenchement, et qu'en position de libération, l'élément de verrouillage (12) libère l'élément à enclenchement (14) de sorte que l'élément à enclenchement (14) peut être libéré de la position d'enclenchement.
dans lequel l'élément à enclenchement (14) comprend au moins une section de déflexion (40) qui est conçu de manière conique, au moins par sections, s'étend de manière oblique par rapport au premier axe de ligne (16) et est élastiquement flexible, ainsi qu'une section à enclenchement (42) qui s'étend pour l'essentiel perpendiculairement au premier axe de ligne (16),
dans lequel l'élément de verrouillage (12) présente une section conique intérieure (32) qui est conçue de manière conique, au moins par sections, et qui, en position de verrouillage, agit contre ladite section de déflexion (40),
dans lequel ledit contre-élément à enclenchement (84) comprend un double cône avec une première section conique (92) conçue de manière conique, au moins par sections, ainsi qu'une deuxième section conique (98) conçue de manière conique, au moins par sections,
dans lequel la première section conique (92) s'élargit jusqu'à un diamètre maximal le long du deuxième axe de ligne (86) et la deuxième section conique (98) est contiguë au diamètre maximal et se rétrécit jusqu'à un diamètre plus petit le long du deuxième axe de ligne (86), et
dans lequel, en position à enclenchement, l'élément à enclenchement (14) s'appuie contre le contre-élément à enclenchement (84), et la section de déflexion (40) s'appuie au moins par sections contre la première section conique (92).

2. Mécanisme de connexion (200) selon la revendication 1, dans lequel l'élément de verrouillage (12) agit contre la section de déflexion (40) en position de verrouillage de telle sorte que sa flexibilité élastique est limitée.

3. Mécanisme de connexion (200) selon l'une quelconque des revendications précédentes, dans lequel l'élément de verrouillage (12) présente une section d'actionnement (26) qui est conçue de manière cylindrique au moins par sections.

4. Mécanisme de connexion (200) selon l'une quelconque des revendications précédentes, dans lequel un élément formant ressort (50) est prévu qui pousse l'élément de verrouillage (12) vers l'élément à enclenchement (14).

5. Mécanisme de connexion (200) selon la revendication 4, dans lequel la première section de ligne (10) est conçue de telle sorte que, en position à enclenchement, elle s'étend au moins par sections dans la deuxième section de ligne (82).

6. Mécanisme de connexion (200) selon l'une quelconque des revendications précédentes, comprenant un contre-élément à enclenchement (84) qui est disposé de manière fixe sur la deuxième section de ligne (82) et est configuré pour s'enclencher, en position d'enclenchement, avec l'élément à enclenchement (14) prévu sur la première section de ligne (10).

7. Mécanisme de connexion (200) selon l'une quelconque des revendications précédentes, dans lequel une section cylindrique (102) qui est conçue de manière cylindrique, au moins par sections, suit la deuxième section conique (98).

8. Mécanisme de connexion (200) selon l'une quelconque des revendications précédentes, dans lequel, en position de verrouillage, la section à enclenchement (42) agit de concert avec la deuxième section conique (98).

9. Mécanisme de connexion (200) selon la revendication 7, dans lequel, en position de verrouillage, la section à enclenchement (42) agit de concert avec la section cylindrique (102).

10. Mécanisme de connexion (200) selon l'une quelconque des revendications précédentes, dans lequel une section de boîtier (90) est prévue qui est disposée sur la deuxième section de ligne (82) ou constitue la deuxième section de ligne (82), dans lequel le contre-élément à enclenchement (84) est disposé sur la section de boîtier (90), dans lequel des éléments de valve hémostatiques (88) sont prévus dans la section de boîtier (90), et dans lequel la conception est telle qu'en position d'enclenchement, la première section de ligne (10) pénètre, au moins par sections, dans la deuxième section de ligne (82) et traverse les éléments de valve hémostatiques (88).
